Europäisches Patentamt

European Patent Office

Office européen des brevets

(19)

(11) Publication number:

**0 391 608**
**A2**

## (12) EUROPEAN PATENT APPLICATION

(21) Application number: **90303382.7**

(51) Int. Cl.5: **C12Q 1/68**

(22) Date of filing: **29.03.90**

(30) Priority: **03.04.89 US 332541**

(43) Date of publication of application:
**10.10.90 Bulletin 90/41**

(84) Designated Contracting States:
**BE CH DE FR GB IT LI NL SE**

(71) Applicant: **MINNESOTA MINING AND MANUFACTURING COMPANY**
**3M Center, P.O. Box 33427**
**St. Paul Minnesota 55133(US)**

(72) Inventor: **Bitner, Rex M. c/o Minnesota Mining and**
**Manufacturing Comp. 2501 Hudson Road**
**P.O.Box 33427**
**St. Paul Minnesota 55133-3427(US)**
Inventor: **Funkenbusch, Eric F. c/o Minnesota Mining and**
**Manufacturing Comp. 2501 Hudson Road**
**P.O.Box 33427**
**St. Paul Minnesota 55133-3427(US)**

(74) Representative: **Baillie, Iain Cameron et al**
**c/o Ladas & Parry Isartorplatz 5**
**D-8000 München 2(DE)**

(54) **Metal oxide supports for nucleic acids.**

(57) Nucleic acids sorbed to metal oxide supports with sufficient strength and in sufficient amounts to enable the resulting compositions of matter to be used for such purposes as hybridizing, labeling, sequencing, and synthesis of nucleic acids. The compositions can optionally also be bound with blocking agent to prevent undesired nucleic acid sorption, e.g., of probe nucleic acids in a hybridization experiment. Preferred compositions are easily prepared and used, versatile, and reusable.

EP 0 391 608 A2

## METAL OXIDE SUPPORTS FOR NUCLEIC ACIDS

### TECHNICAL FIELD

The present invention relates to supports useful for the immobilization of nucleic acids. In another aspect, the present invention relates to metal oxides, including hydrous metal oxides, for use as supports for biological molecules. The present invention also relates to attachment of labels, such as enzyme labels, to nucleic acids, e.g., by formation of conjugates that include nucleic acids and labels.

### BACKGROUND OF THE INVENTION

An important technique used in analysis of nucleic acids is that of hybridization, e.g., hybridization of DNA to DNA, or DNA to RNA. One type of nucleic acid hybridization technique involves immobilization of DNA onto a solid phase, such as described by Southern, et al., J. Mol. Biol., 98:503-517 (1975). This technique, known as "Southern blotting", involves first the separation of DNA molecules from one another on the basis of their electrophoretic mobility; followed by immobilization of the resultant separated DNA molecules onto a support or substrate such as a filter (nitrocellulose, diazotized paper, and nylon are widely used). This is followed by hybridization of a labeled DNA probe or probes to the corresponding immobilized DNA sequences. RNA can also be electrophoretically separated and blotted in an analogous manner, in a technique referred to as "Northern blotting".

If the labeled probe contains a nucleotide sequence that is complementary to an immobilized DNA sequence, the probe will anneal by hydrogen bonding (i.e., "hybridize") to the immobilized sequence. The presence of hybridization can be determined and, in turn, correlated with the presence of certain DNA sequences, e.g., genes or parts of genes, in either the source providing the probe molecules or the source that had been electrophoretically separated.

Immobilized nucleic acids have been used in other procedures as well, such as sequencing procedures, and procedures for synthesis of nucleic acids. As an example of the latter use, immobilized RNA has been used to facilitate production of complementary DNA (cDNA) by reverse transcriptase. See, e.g., Stoflet, et al., Science, 239:491-494 (1988).

A variety of supports have been described and used for immobilizing nucleic acids, e.g., nitrocellulose, diazotized paper, hydroxylapatite, nylon, and magnetic dynospheres (see, e.g., Matthews, et al., Analyt. Biochem., 169:1-25 (1988)). Commercially available supports include supports made of polystyrene, polypropylene, acrylic, and latex microparticles. Other available supports are "Sephacryl S-500", cellulose-magnetic beads known as "CNBr-Sepharose 4B", and "Sephadex G-50", each available from Pharmacia Fine Chemical Co. (Uppsala, Sweden), and "Whatman 541" paper (Whatman International Ltd., Maidstone, England).

There are a number of inherent difficulties associated with use of many of these materials however, including at the outset, the need to identify and select different supports depending on the intended use. For instance, DNA immobilized on a nitrocellulose filter can generally only be used in a single hybridization, and DNA generally must be baked onto the filter in order to irreversibly bind the DNA to the nitrocellulose. Moreover, nitrocellulose does not bind nucleic acids well in low ionic strength buffers, thereby limiting its use.

Whereas diazotized paper can be reused, it is difficult to prepare and tends to be too fragile for extensive, repeated use. Similarly, nylon can be reused, and is the most widely used support for Southern blotting. Nylon however generally does not provide optimal binding of DNA unless a subsequent treatment is included in the spreparation procedure, such as ultraviolet irradiation in a high salt buffer. (See, e.g., Khandjian, Biotech., 5:165-167 (1987)).

Certain patents describe supports that may contain some metal oxides, as useful for binding nucleic acids. U.S. Pat. No. 3,652,761, for instance, describes antigens, which are described as including nucleic acids, covalently bound through an intermediate silane coupling agent to hydroxy containing inorganic carriers.

Additionally, U.S. Pat. No. 4,672,040, describes magnetic particles having a metal oxide magnetic core generally surrounded by a coat of a polymeric silane, and the use of such particles, inter alia, for carrying out nucleic acid hybridization, using nucleic acid coupled to the silane.

U.S. Pat. No. 4,713,326 describes solid supports capable of binding nucleic acids upon irradiation and having a substrate, a photochemically reactive nucleic acid-binding ligand and a divalent radical chemically linking the substrate and the ligand.

U.S. Pat. No. 4,748,121, describes the use of porous, silica-rich, glass fibers for immobilization of biochemically active materials, which materials are broadly defined to include nucleic acids. The fibers are described as being formed from a composition containing major amounts of both $B_2O_3$ and $SiO_2$, together with minor amounts of alkali metal oxides, $ZrO_2$, and $Al_2O_3$. The initial combined amount of $ZrO_2$ and $Al_2O_3$ in the '121 patent does not appear to exceed about 9 weight percent however. Biochemically active material is attached to the fibers by absorption or by covalent binding with a linking agent.

What the prior art has not taught, but what the present invention provides, is an economical, durable, and reusable material for the immobilization of nucleic acids that is easy to use and versatile enough to be provided in a variety of structures and forms.

## SUMMARY OF THE INVENTION

The present invention provides a novel composition of matter, as well as a method for preparing and a method for using such a composition, wherein the composition comprises;

(a) a support comprising an amount of metal oxide sufficient to sorb nucleic acid, and

(b) nucleic acid sorbed to at least a portion of the available surface of the support in a manner such that the nucleic acid substantially retains biological accessibility and reactivity.

Preferably the composition further comprises blocking agent, bound to the remainder of : surface of the support in a manner that substantially precludes the ability of the support to sorb additional nucleic acid.

Preferred compositions can; (1) be provided in a variety of forms, (2) be sterilized, (3) sorb nucleic acid as well as bind blocking agent, and (4) be reused, either by removing only the hybridized nucleic acid and rehybridizing the sorbed nucleic acid, or by removing the sorbed nucleic acid as well, and re-sorbing new nucleic acid.

Surprisingly, supports of the present invention sorb nucleic acids with sufficient tenacity to maintain them in that immobilized state even under demanding conditions, yet in a manner that allows the nucleic acids to substantially retain their biological accessibility and reactivity, e.g., remain free or otherwise available for further chemical and/or physical interactions, such as hybridization.

Compositions of this invention can be prepared in a variety of forms, e.g., as particles such as colloidal particles, free particles, and porous spherules, or in the form of fibers or solid monolithic bodies. Forms, such as particles, can be used in a variety of ways such as in a liquid, in a coating, or in a composite structure, e.g., integrated with a porous web.

Compositions can be used for a variety of applications, e.g., as nucleic acid supports during hybridization, for labeling nucleic acids, for sequencing nucleic acids, and/or for nucleic acid synthesis.

Moreover, preferred compositions can be reused, either by removing the hybridized nucleic acid and using the originally sorbed nucleic acid again, or by removing both the sorbed and hybridized nucleic acids and using the support to re-sorb new and/or different nucleic acid. Both attributes provide great flexibility, e.g., enabling these compositions to be used and then reused in biological reactors or columns, or for sequential processes.

Compositions of this invention provide an optimal combination of ease of preparation, sorption affinity, sorption capacity, shelf-life stability and stability in use, versatility, reusability, and cost.

## DETAILED DESCRIPTION

### 1. Compositions

The present invention provides a novel composition of matter and a method for preparing such a composition, the composition comprising;

(a) a support comprising an amount of metal oxide sufficient to sorb nucleic acid,

(b) nucleic acid sorbed to at least a portion of the available surface of the support in a manner such that the nucleic acid substantially retains biological accessibility and reactivity.

Preferably the composition further comprises blocking agent, bound to the remainder of the surface of the support in a manner that substantially precludes the ability of the support to sorb additional nucleic acid.

While not intending to be bound by theory, it is believed that the nucleic acid is immobilized to metal oxide by a "sorption" process, e.g., involving chelation of functional groups of the nucleic acid by the metal oxide. The word "sorb" and inflections thereof, as used herein, refers to any adsorption or absorption process, whether physical and/or chemical in nature, that results in the non-covalent binding of nucleic acid to metal oxide, in a manner that allows the nucleic acid to be used for its intended purpose.

## 1.a. Supports

A "support" of the present invention has a surface that is physically accessible to nucleic acid and that contains an amount of metal oxide sufficient to sorb nucleic acid. A support can therefore be a homogeneous structure, such as an entire particle, fiber, or monolithic body containing a sufficient amount of metal oxide. A support can also be used to refer to those portions of a heterogeneous structure that contain sufficient metal oxide, such as a metal oxide-containing layer of a coated structure, or metal oxide-containing islands or areas within a larger, non-metal oxide structure.

Metal oxide is present in at least the surface of the support in a substantial amount, i.e., an amount sufficient to sorb the desired amount of nucleic acid for the intended purpose of the composition. Metal oxide is preferably present as at least about 50%, or more, by weight of the elements comprising the available surface of the support. More preferably, metal oxide is present as at least 70% by weight, and particularly preferred are supports in which metal oxide is present as at least about 80% by weight of the elements comprising the available surface of the support. Most preferred are supports made up substantially entirely, e.g., greater than 99%, or at least about 90% by weight, of metal oxide. The amount of metal oxide present in a homogeneous support, i.e., a support wherein the amount of metal oxide is constant throughout, is preferably determined by the Inductively Coupled Plasma ("ICP") emission spectroscopy method, as described in Inductively Coupled Plasma Emission Spectroscopy, Parts I and II, P.W.J.M. Boumans (ed.), John Wiley and Sons, New York (1987), the disclosure of which is incorporated herein by reference.

The amount of metal oxide actually present at the surface of a support, e.g., a heterogeneous support wherein the amount of metal oxide may vary between the surface and sub-surface or other regions, can be determined by any suitable method capable of determining elemental composition by surface analysis. For purposes of the present invention, the amount can be determined by X-ray photoelectron spectroscopy ("XPS"), as described in Chapter 4 of "Methods of Surface Analysis" (in Vol. 1 of Methods and Phenomena - Their Applications in Science and Technology, A. Czanderna, ed., Elsevier Scientific Publishing Co., 1975), the disclosure of which is incorporated herein by reference.

In XPS, a focused X-ray beam irradiates a specimen or sample, producing photoelectrons that are then characterized to determine their energy and intensity. Energies of the photoelectrons are specific to particular elements and their chemical states. Generally, XPS is a non-destructive technique that can provide an analysis of the outermost 30 to 100 Å of the specimen surface, and is sensitive to all elements in the periodic table except hydrogen and helium, with detection limits for most species in the 0.1 to 0.5% atomic concentration range. Area of surface analyzed is generally adjustable from 150 microns to 3 mm diameter.

Suitable metal oxides of the present invention exhibit an optimal combination of such properties as strength of sorption, sorption capacity with respect to nucleic acids, and the ability to thereafter be "blocked", as defined herein, by blocking agents. As used herein, the term "metal oxide" shall refer collectively to metal oxides and hydroxides as well as hydrous metal oxides and hydroxides. The word "hydrous" as used herein refers to metal oxide or hydroxide surfaces containing physically and/or chemically adsorbed water, and will be used in parentheses herein to indicate the optional presence of such water. "Strength" of sorption, as used herein, refers to the ability of sorbed nucleic acid to remain sorbed to the metal oxide for purposes of the intended use of the resulting composition, e.g., under the conditions of a hybridization experiment. "Sorption capacity" of the metal oxide, refers to the ability of the particular metal oxide support to sorb enough nucleic acid, per unit weight or surface area of the support, for the intended use of the resulting composition.

Examples of suitable metal oxides include, but are not limited to, the (hydrous) oxides and hydroxides of: magnesium, calcium, titanium, manganese, cobalt, nickel, zinc, yttrium, and lanthanum.

Examples of preferred metal oxides include, but are not limited to, the (hydrous) oxides and hydroxides of aluminum, iron, zirconium, and hafnium. Particularly preferred are the (hydrous) oxides and hydroxides of zirconium. When it is desired that supports of the present invention provide magnetic properties, preferred metal oxides include iron, cobalt, and nickel.

Preferably the available surface area of a support is greater than about 1 $m^2/g$ metal oxide, and particularly preferred are supports having surface areas of greater than about 5 $m^2/g$ metal oxide. Surface area can be determined in any suitable fashion. For purposes of the present invention, surface area is determined by nitrogen absorption, as described in Absorption Surface Area and Porosity, S.J. Gregg and K.S.W. Sing, Academic Press, London and New York (1967), the disclosure of which is incorporated by reference.

Compositions of the present invention have nucleic acid sorbed to at least a portion of the available surface of the support in a manner that substantially retains biological accessibility and reactivity of the nucleic acid. The word "substantially", as used in this context means that the nucleic acid can be used for its intended purpose, be it hybridization, labeling, sequencing, or the like.

### 1.b. Nucleic Acids

Nucleic acid is sorbed to at least a portion of the available surface of the support. The term "available surface", as used in this sense, refers to the support surface that is physically accessible for sorption of nucleic acid. For instance, for a support such as a metal oxide particle entrapped in a web or embedded in a coating, as described more fully below, the available surface of the support itself (i.e., particle) is the surface not physically occluded from nucleic acid sorption, e.g., by contact with other particles or with the web or coating itself. Similarly, for a support such as a porous particle, the available surface would include any surfaces of pores large enough to be accessible to nucleic acid.

Supports of the present invention are able to sorb lengths (based on numbers of base pairs) of nucleic acids useful for such purposes as hybridization experiments. Generally such nucleic acids will be between about 200 to about 10,000 bases in length. Nucleic acid probes useful for hybridizing with sorbed nucleic acids will generally be between about 10 bases to about 30,000 bases in length.

The term "nucleic acid", as used herein, broadly refers to any molecule containing sufficient amounts (e.g., regions) of polynucleotides to enable the molecule to sorb to a support, including, for example, single- or double-stranded DNA or RNA, oligonucleotides, and nucleoproteins. Surprisingly, strong sorption can be achieved in compositions of the present invention even with double stranded DNA, i.e., where nucleoside groups are oriented predominantly towards the interior of the DNA. This suggests that phosphate groups of the DNA backbone may play a significant role in the sorption of nucleic acids to metal oxides.

### 1.c. Blocking Agent

Preferably compositions of the present invention also have blocking agent bound to the remainder of the surface of the support in a manner that substantially precludes the ability of the support to sorb additional nucleic acid. By "the remainder" of the surface of the support is meant the surface that is accessible to but not already sorbed with nucleic acid. The term "blocking agent", as used herein refers to a material, e.g., compound or molecule, capable of binding to the remainder of the available surface of the support to prevent sorption of probe nucleic acid directly to the support. The word "bound" as used herein refers to the binding of blocking agent in sufficient amounts and with sufficient strength to enable the support to be used for its intended purpose, e.g., by preventing unwanted sorption of probe DNA sequences to such surfaces in the course of a hybridization experiment.

Suitable blocking agents exhibit an optimal combination of such properties as binding strength, binding capacity, and cost. Suitable blocking agents include, but are not limited to, organic or inorganic molecules providing phosphate, phosphonate, pyrophosphate, carboxylate, sulfate and fluoride groups. Preferred blocking agents include, but are not limited to, potassium phosphate, potassium pyrophosphate, potassium fluoride, ethylenediaminetetraacetic acid ("EDTA"), and lauryl sulfate.

### 2. Forms of Supports

Compositions of this invention can be prepared using supports in a variety of forms, e.g., as particles such as colloidal particles, free particles, and porous spherules, or in the form of fibers or solid monolithic bodies. Particles can be used in any size, e.g., from submicron size to particles many millimeters in size, and can be used in a variety of ways such as in suspension, in a coating, or in a composite structure, e.g., integrated with a porous web.

2.a. Particles

The term "colloidal particles", as used herein, refers to particles dispersed, or capable of being dispersed, in a colloidal suspension. "Colloidal suspension" as used herein refers to a form intermediate between true solution (wherein a solute is present in ionic or molecular dimensions) and bulk solute. Generally such particles have an average particle size of on the order of a micron or less. Colloidal particles are preferred in that they do not aggregate in water, and the small size of colloidal particles provides a high surface to volume ratio, which in turn tends to provide these particles with a high sorption capacity.

Colloidal particles sorbed with nucleic acid, with or without probe nucleic acid hybridized thereto, can be separated from aqueous medium by a variety of methods, such as flocculation, precipitation, evaporation (such as "rotovaping"), or sedimentation, as by centrifugation. Colloidal particles of many metal oxides described herein are commercially available, e.g., from Nyacol, Inc. (Ashland, MA) and Nalco, Inc. (Naperville, IL), or can be prepared by methods known in the art. See, e.g., Weiser, H.B., Inorganic Colloid Chemistry, Vol 2, Wiley and Sons, N.Y. 1949, the disclosure of which is incorporated herein by reference.

Free metal oxide particles, e.g., in dry powdered form, are known and are useful in the present invention, such as particles having a average size of on the order of a micron or more in their smallest dimension. Although such particles cannot generally be maintained in colloidal suspension, they are particularly useful when incorporated into a matrix or other composite structure as described below.

Free particles of many metal oxides are commercially available, e.g., as powders, from a variety of sources, such as Sigma Chemical Co., St. Louis, MO, and Aldrich Chemical Co., Milwaukee, WI.

Preferred particles of the present invention include porous spherules, i.e., spherical particles, having an average particle diameter between about one-half and about 500 microns, and preferably between about one and about 100 microns. Such particles can be prepared by a variety of methods, such as those described in U.S. Patent Nos. 4,010,242 and 4,138,336, as well as in copending application U.S. Serial No. 151,819 filed February 3, 1988, the disclosures of each of which are incorporated herein by reference.

2.b. Fibers and Monolithic Bodies

In addition to the use of particles, supports of the present invention can also be prepared in the form of fibers. Fibers made of a variety of metal oxides have been made and are commercially available. See, e.g., Inorganic Fibers and Composite Materials, EPO Applied Technology Series, Vol. 3, Pergamon Press, 1984.

In addition to the use of particles and fibers, supports of the present invention can also be prepared in the form of monolithic bodies, which can be either porous or dense. The term "monolithic bodies", as used herein refers to uniform, solid bodies larger than particles, e.g., having physical dimensions large enough to enable them to be handled and used directly. Monolithic bodies are generally less preferred than porous spherules, however, since the pore size of such bodies may be difficult to control, and the monolithic bodies tend to exhibit a less favorable surface area to volume ratio.

Preparation of monolithic bodies can be accomplished by a variety of means, see e.g., Kingery, et al., Introduction to Ceramics, Ed. 2, Wiley and Sons, New York, NY 1976, the disclosure of which is incorporated by reference. For instance, such bodies can be prepared by compacting a suitable metal oxide powder into the desired shape, e.g., using a binder, followed by heating to an elevated temperature to induce sintering (i.e., bonding of the particles to each other), while at the same time maintaining suitable porosity. It is preferred that the bodies be porous so as to have a higher surface area and therefore a higher sorption capacity.

3. Uses of Supports

Supports of the present invention can be used in a variety of ways, e.g., particles can be used in suspension, in a coating, or in a composite, e.g., integrated with a porous web.

3.a. Colloidal Particles

6

Colloidal particles can be used directly to prepare compositions, e.g., by sorbing nucleic acid, blocking, and hybridizing the sorbed nucleic acid while the particles are in suspension, as described more fully below. Use of the resultant compositions in suspension tends to optimize the kinetics of hybridization. Additionally use of such particles or compositions in suspension facilitates separation of the particles from solution at any desired point in time, e.g., by the methods described more fully below.

## 3.b. Coatings

Supports of the present invention can also be prepared as a coating of metal oxide on a suitable substrate, e.g., an organic or inorganic substrate, such as a polymer, glass, or metal substrate. Metal oxide can be coated in a variety of ways, e.g., from solution or from a vapor phase. Moreover, metal oxide can be coated as a colloidal or powdered metal oxide, or as a precursor, such as a metal salt or metal alkoxide which can be converted to the metal oxide by suitable treatment (e.g., by heating or exposure to water). Preferred coatings are thin films (e.g., down to monomolecular thickness coatings), porous thick films, and membranes.

## 3.c. Composite Structures

Supports suitable for use in compositions of the present invention can also be prepared and used as part of a composite structure, e.g., incorporated into, or otherwise held by, a carrier such as a matrix or membrane. Suitable carriers exhibit an optimal combination of such properties as in situ accessibility of the metal oxide to both sorbed and probe nucleic acid, porosity, wettability, physical durability, chemical inertness, dimensional stability, and cost. Examples of suitable carriers include, but are not limited to, polyethylene, nylon, and polyester membranes.

An example of a preferred membrane is polytetrafluoroethylene ("PTFE"), as described in U.S. Patent No. 4,153,661, the disclosure of which is incorporated herein by reference. Such membranes provide exceptional loading capacity, chemical stability, and versatility for purposes of their use in the present invention.

Particularly preferred are composite structures wherein porous $ZrO_2$ spherules are incorporated into a PTFE web, such as those prepared according to the method described in U.S. Patent No. 4,810,381, the disclosure of which is incorporated herein by reference. Such composite structures exhibit good blotting capacity, are stable in strong acid and base, and are stable at elevated temperatures. Since such structures can be easily handled, cleaned, and sterilized by immersion in strong acid or base and/or by exposure to elevated temperature, they are reusable without exhibiting substantial damage.

Advantages of the preferred composite structures include elimination of the need for pre- or post-treatment of the support surface to obtain strong sorption of nucleic acids. The PTFE membrane acts essentially as a physical trap, preventing dislocation of metal oxide particles, while not interfering with the surface sorption characteristics of the particles. The structures can be regenerated and reused (e.g., rehybridized) numerous times, due to their inherent physical toughness, good resistance to temperature, and to strong acid and base. The structures are also compatible with strongly ionic solutions, and are chemically inert in the temperature range of intended use.

An added advantage of the preferred composite structures of the present invention is that nucleic acids can be sorbed to the metal oxide support at any suitable time with respect to formation of the composite structure itself, e.g., prior to, during, or after formation of the composite structure containing the support.

## 4. Preparation and Use of Compositions

The method of preparing a composition of the present invention involves the steps of
(a) providing a support comprising an amount of metal oxide sufficient to sorb nucleic acid,
(b) sorbing nucleic acid to at least a portion of the available surface of said support in a manner such that the nucleic acid substantially retains biological accessibility and reactivity.

Preferably the method also comprises an additional step of binding blocking agent to the remainder of the available surface of said support in a manner that substantially precludes the ability of the support to sorb additional nucleic acid.

Sorption can be accomplished in any suitable manner. For instance, nucleic acid sorption can be accomplished in an aqueous sol of the colloidal particles by contacting the nucleic acid with the particles directly in suspension, followed by blocking and hybridization, without the suspension becoming unstable. As described more fully below, the amount of nucleic acid sorbed to the support, as well as the total sorption capacity and sorption strength can be evaluated in a variety of ways using techniques familiar to those skilled in the art, such as by the use of radiolabeled nucleic acids.

With supports such as those in the form of metal oxide coatings or particles incorporated into a composite structure, sorption can be accomplished by contacting the support with a solution that contains nucleic acid under conditions that allow the solution to wet the support, thereby providing aqueous contact between the support and nucleic acid.

Compositions can be used for a variety of applications, e.g., as nucleic acid supports during hybridization, for labeling nucleic acids, for sequencing nucleic acids, or for nucleic acid synthesis.

Nucleic acids immobilized by sorption onto the preferred composite structures, such as the preferred $ZrO_2$-PTFE structure discussed above, are preferred for use in nucleic acid hybridization. One example of such an assay is called a DNA (or "Southern") blot.

In a Southern blot, DNA is cut into various sized fragments, usually through the use of DNA restriction enzymes, and the resultant fragments are separated into a size distribution, using gel electrophoresis. Separation takes place inside a gel matrix, frequently an agarose gel or an acrylamide gel, in which the DNA fragments migrate under the influence of an electric current. DNA fragments migrate toward the positive electrode, and because the smaller-sized fragments migrate faster through the gel matrix, the result is a separation of the fragments into a size distribution within the gel matrix.

DNA fragments in the gel matrix are transferred to the preferred $ZrO_2$-PTFE composite structure, usually by electrophoresis ("electroblotting"), or by eluting the DNA by passing an aqueous solution directly through the gel and into the composite structure. The DNA is carried by facilitated diffusion, in the liquid phase, into the composite structure where the DNA is able to sorb to the metal oxide surface of the support.

Once the DNA has sorbed to the support, the surface is preferably blocked with blocking agents such as those described previously. Blocking substantially prevents further binding of nucleic acid molecules, that is, any remaining available binding sites are made unavailable to further binding (e.g., of probe DNA); but does not interfere with the intended use of the previously sorbed molecules.

After blocking, the sorbed molecules can be used for hybridization. For example, sorbed DNA or RNA molecules can be tested for sequence homology to a particular nucleic acid sequence using a "DNA hybridization" procedure, in which a labeled DNA sequence is reacted with the sorbed nucleic acids. The labeled sequence ("probe") will hybridize to homologous DNA sorbed to the support. In turn, the presence (in the sorbed nucleic acids) of sequences homologous to the "probe" can be correlated with the presence or absence of certain genes or nucleic acid sequences in the probe molecules, in a manner that will be familiar to those of ordinary skill in the art.

At the conclusion of a particular hybridization procedure, using a particular DNA sequence as a "probe", the composite structure can be cleaned, e.g., by immersion for on the order of 3-5 minutes in boiling water, in order to remove nucleic acid hybridized to the sorbed DNA, without affecting the sorption of the DNA to the support surface. Once cleaned with boiling water, DNA sorbed to the support can be hybridized with a second, optionally different, labeled DNA probe. Similarly, the composite structure can again be cleaned with boiling water. The structure can be repeatedly reused in this manner.

As an important attribute of preferred composition of the present invention sorbed nucleic acids themselves can also be removed from supports, e.g., by exposing the membranes to very high pH, such as at least pH 12, or preferably at least pH 13. Preferably such exposure is also at elevated temperature, e.g., on the order of 80° C, for a period of about 10 minutes or more, and the exposure to high pH and elevated temperature is repeated one or more times to ensure complete desorption.

Quantitation of a particular nucleic acid sequence in a biological sample can be achieved with compositions of the present invention, e.g., using dot or slot blotting techniques (See, e.g., Schuster, et al, J. Infect. Dis., 154:309-314, 1986, the disclosure of which is incorporated herein by reference), where a known amount of DNA is sorbed in the form of a dot, or in the form a linear or curved slot, onto a support, e.g., within a composite structure.The amount of DNA probe hybridizing to the sample DNA is proportional to the amount of the homologous sequence present in the sample.

Compositions of the present invention can also be used to simultaneously bind to both a nucleic acid and to a label, thereby providing the ability to form a stable conjugate between the label and nucleic acid. Structurally, such conjugates are analogous to "spot-welding" the nucleic acid to the label, since generally the label, such as a protein, will be much larger than either the nucleic acid portion itself or the metal oxide support connecting the two.

Such conjugates can be made in a variety of ways depending upon the desired degree of labeling required, for instance, by either first binding the label to metal oxide particles, followed by mixing with nucleic acid, or by mixing metal oxide particles with a mixture of nucleic acid molecules and labels. Such labels have previously been attached directly to nucleic acids by chemical means, e.g., for use as detection means in assays such as hybridization assays. See, e.g., U.S. Pat. No. 4,729,947, the disclosure of which is incorporated herein by reference. Typical protein labels include antibody, antigen, enzyme, avidin, and strepavidin labels. Enzyme labels such as alkaline phosphatase, horseradish peroxidase, bacterial luciferase, and firefly luciferase have been used for such purposes, as have radioactive, fluorescent, magnetic, antigenic, and chemically catalytic labels.

Compositions of the present invention can also be used in reactions that determine the sequence of the sorbed nucleic acid. This can be done by using the sorbed sequences as templates in a sequencing reaction, for example, as templates in a dideoxy, chain elongation reaction, as described for example, in Saiki, R.K. et al, Science, 239:487-491, 1988, the disclosure of which is incorporated herein by reference. Sorbed RNA transcripts can also be used as templates for reverse transcriptase, which is an enzyme that makes complementary DNA copies of the RNA sequence. These DNA copies can then be used in a sequencing reaction (See, e.g., Stoflet, E.S. et al, Science, 239:491-494, 1988, the disclosure of which is incorporated herein by reference).

Preferred compositions of the present invention can be exposed to traditional sterilization conditions, for example by exposure to steam and/or heat, or high pH, without significant degradation. This is also true for preferred composite structures, e.g., $ZrO_2$ spherules that have been incorporated into a membrane, or coated onto a surface, provided that the membrane or coated material can also tolerate the sterilizing conditions. $ZrO_2$ spherules incorporated into PTFE membranes, as described in the preferred embodiment, can be exposed to traditional sterilization conditions, such as autoclaving, without significant degradation.

Supports useful in the present invention can be made and sold that are useful for preparing a wide variety of compositions, e.g., when sorbed with different types of nucleic acid. Such supports can be packaged and sold in the form of a kit, e.g., together with instructions for sorbing, and optionally blocking, the supports.

Objects and advantages of this invention are further illustrated by the following EXAMPLES, but the particular materials and amounts thereof recited in these examples, as well as other conditions and details, should not be construed to unduly limit this invention.

## EXAMPLES

## EXAMPLE 1

## DNA Sorption to Metal Oxide Powder Surfaces

A qualitative evaluation of binding of nucleic acid to metal oxide supports was obtained by the following procedure: Buffer solutions containing varying concentrations of DNA (phage lambda DNA, cut with restriction enzyme Bst EII) were aliquoted into polypropylene micro test tubes (Bio Rad catalog No. 223-9503, Richmond, CA). A typical volume was about 40 $\mu$l of solution per tube, and DNA concentrations included 0.005, 0.01, 0.05 and 0.1 $\mu$g/$\mu$l.

Various metal oxides in a variety of forms (powder, spherules, etc.) were then added in uniform amounts (typically 25 mg metal oxide per tube) to individual DNA containing tubes. The DNA solution and metal oxide were vigorously mixed for 60 seconds, and the tubes were centrifuged for 2 minutes at 5000 xg to pellet the metal oxide. 20 $\mu$l of supernatant was removed from each test tube, and subjected to electrophoresis in a 1% agarose gel containing 5 $\mu$g/ml of ethidium bromide.

The concentration of DNA present in the aliquot of supernatant was compared to the concentration initially present in 20 $\mu$l of the original solution. A qualitative evaluation was made for DNA sorption by the metal oxide by comparing the color of the resultant solution. The more intense the color, the more unsorbed DNA present in solution, and in turn the less DNA sorbed to the support.

Samples of the following metal oxides were subjected to the screening procedure and found to possess adequate sorptive capacity for purposes of the present invention: CaO, $Y_2O_3$, $ZrO_2$, $HfO_2$, $La_2O_3$, $TiO_2$,

MgO, $MnO_2$, alpha-FeOOH, CoO, and $Al_2O_3$.

Supports screened were those already on hand or otherwise readily available, i.e., they were not optimized for purposes of this EXAMPLE. It should be noted therefore that low sorption for any particular support may simply have been the result of low surface area for a particular sample, and was not necessarily indicative of the inherent sorption strength of the particular metal oxide. This screening procedure, however, helps one to assess the suitability of a particular metal oxide sample for purposes of the present invention, both in terms of the inherent binding strength of the metal oxide per se, together with the surface area of the particular support.

EXAMPLE 2

DNA Sorption and Hybridization Assay

$ZrO_2$ powder, obtained from Sigma Chemical Company ((catalog number Z-2500), St. Louis, MO) was used as a support for DNA sorption. The following procedure was performed at 22 degrees C: 2 μg of either phage lambda DNA or plasmid B. subtilis pUB110 DNA (i.e., not homologous with phage lambda DNA) was mixed in a polypropylene micro test tube with 25 mg of $ZrO_2$ powder in 150 μl of autoclave-sterilized, distilled, deionized water that had been purified using a Millipore Corporation (Bedford, MA) "Milli Q" system. The DNA was allowed to sorb to the $ZrO_2$ surface for 30 minutes at 22 degrees C. The tube was centrifuged and the supernatant removed.

To block the remaining surface of the support having DNA sorbed thereto, the resulting pellet was resuspended in 200 μl of 5% potassium phosphate (pH 7.0) solution in a test tube. The tube was centrifuged, the supernatant removed, and the pellet washed a second time with 200 μl of 5% potassium phosphate (pH 7.0). The tube was again centrifuged, the supernatant removed, and the resultant pellet was resuspended in a test tube in 200 μl of autoclave-sterilized, distilled, deionized water. The pellet-containing tube was centrifuged, the supernatant removed, and the pellet resuspended in 200 μl of autoclave sterilized, distilled, deionized water.

The $ZrO_2$ powder sorbed with DNA was centrifuged, the supernatant removed, and the pellet was resuspended in a test tube containing 150 μl of a solution having a final concentration of: 10X "Denhardts" hybridization buffer (50X Denhardts is 1% polyvinylpyrrolidone, 1% BSA fraction V, and 1% Ficoll), 5X Sodium Citrate Buffer ("SSC") (1X SSC is: 0.88% NaCl, 0.44% sodium citrate), 50mM Tris[hydroxymethyl]-aminomethane (pH 7.5), 0.1% sodium pyrophosphate, 1% sodium dodecyl sulfate ("SDS"), and 500 μg/ml salmon sperm DNA.

To achieve prehybridization equilibrium, the tube was incubated for 3 hours at 55 degrees C, then centrifuged and the supernatant removed. The pellet was resuspended in a test tube containing 100 μl of "hybridization solution" having a final concentration of: 50% formamide (Aldrich Chemical Company, catalog number 27,054-7, Milwaukee, WI), 10% dextran sulfate, 5X SSC, 1X Denhardts. Lambda DNA and plasmid pUB110 DNA were previously radioactively labeled with $^{32}$P labeled nucleotides, using a "nick-translation" kit, from Amersham Corporation (Arlington Heights, IL). 10 μl of $^{32}$P labeled lambda DNA solution, or $^{32}$P labeled pUB110 DNA, was added to 150 μl of 10 mg/ml salmon sperm DNA. The tube was placed in boiling water for 5 minutes, and then placed in an ice bath for 5 minutes. An aliquot of the labeled DNA probe was then added to the tube containing the DNA sorbed to $ZrO_2$ powder in hybridization solution, as described above.

To enable hybridization to occur, the mixture was incubated at 42 degrees C for 12 hours, with slight agitation to ensure mixing. The tube was centrifuged, the supernatant removed, and the pellet washed by resuspending in a test tube containing 300 μl of 2X SSC/0.1% SDS. This tube was incubated 15 minutes at 22 degrees C. The tube was centrifuged, the supernatant removed, the pellet resuspended in a test tube containing 300 μl of 2X SSC/0.1% SDS for 15 minutes at 42 degrees C. The tube was centrifuged, the supernatant discarded, the pellet resuspended in a test tube containing 300 μl of 0.1X SSC/0.1% SDS, and the tube was incubated for 30 minutes at 42 degrees C. The tube was centrifuged, the supernatant removed, the pellet resuspended in a test tube containing 300 μl of 0.1X SSC/0.1% SDS and incubated 30 minutes at 42 degrees C. The tube was centrifuged, the supernatant removed, and the radioactivity in the pellet counted using a scintillation counter.

When the initial amount of radiolabeled DNA added was about 100,000 counts per minute, the amount of radioactivity remaining at the end of the hybridization would be at least 5 times greater in those tubes where the same (i.e., homologous) DNA sequences are both sorbed to the $ZrO_2$ powder and are used as the radiolabeled probe, compared to tubes where non-homologous DNA is sorbed. For example, a typical experiment produced the following results: Averaging triplicate samples, 84,000 counts per minute (CPM) were added initially. Hybridization using homologous DNA sequences resulted in 5000 CPM being hybridized to the sorbed DNA, whereas the hybridization of non-homologous DNA sequences resulted in values of about 350 CPM hybridized to the sorbed DNA. In other words, homologous DNA probes were effectively hybridized, as compared to non-homologous probes, using a composition of the present invention.

## EXAMPLE 3

### Nucleic Acid Binding to Zirconia Spherules

Zirconia spherules were prepared in the manner described in United States Patent application (U.S. Serial No. 151,819, filed February 3, 1988). Peanut oil (3 liters) was placed in a 4 liter beaker and heated to 90 degrees C. A mechanical agitator was inserted and the peanut oil was vigorously stirred. One hundred grams of Nyacol™ Zr 95/20, a colloidal $ZrO_2$ suspension available from Nyacol Inc. (Ashland, MA) and containing 20%, by weight, of $ZrO_2$ particles (with a stated particle size of about 95 nm in diameter), was sprayed into the peanut oil through an aerosol atomizer. After approximately 30 minutes, the batch was filtered through a No. 54 Whatman filter. Approximately 17 g of solids were recovered, which were predominantly spherules having a diameter of less than 30 microns. The recovered spherules were heated to a temperature of 600 degrees C for 6 hours. The surface area of the recovered spherules was determined to be 34 $m^2/gm$ by nitrogen adsorption.

The $ZrO_2$ spherules were used in the DNA sorption procedure described in EXAMPLE 2, to form compositions which were then hybridized as described in EXAMPLE 2. In a typical experiment 4.0% (by CPM) of homologous pTM624 probe DNA hybridized with sorbed pTM624, compared to 0.2% of lambda DNA probe hybridized (essentially background), and in turn 1.9% of lambda DNA probe hybridized with sorbed lambda DNA, as opposed to 0.2% background. These results correlate well with those described in EXAMPLE 2.

## EXAMPLE 4

### Nucleic Acid Sorption and Hybridization with Various Metal Oxides

Sorption of phage lambda DNA and hybridizations were performed (according to EXAMPLE 2) using various metal oxide supports, the results of which are listed below in TABLE 1. DNA hybridization was performed with E. coli phage lambda DNA (homologous) and plasmid pTM624 DNA (non-homologous).

$ZrO_2$ sol was obtained as "Nyacol Zr 100/20" colloidal $ZrO_2$ (20% $ZrO_2$, pH 3, particle size approximately 100 nm) from Nyacol, Inc.

Alpha FeOOH sol was prepared as approximately 4 weight percent, pH 3, particle size approx. 100 - 200 nm.

$Al_2O_3$ sol was prepared by dispersing Dispural™ alpha alumina monohydrate powder (Condea) with nitric acid at an Al to $HNO_3$ ratio of approximately 8.75 to 1, to obtain 4 weight percent $Al_2O_3$, pH 3, and a particle size of approximately 55-85 nm.

CoO was obtained as a powder from City Chemical Corporation (New York), Cat. No. WB893.

$HfO_2$ powder was obtained from Aldrich Chemical Co., Cat. No. 20,211-8.

TABLE 1

| DNA Hybridization | | | |
|---|---|---|---|
| metal oxide support | $^{32}$P labeled probe | CPM on sol/pTM624 | % of total counts added |
| ZrO$_2$ sol | pTM624 | 5429 | 6.4 |
| | pTM624 | 4566 | 5.4 |
| | lambda | 360 | 0.4 |
| | lambda | 320 | 0.4 |
| alpha FeOOH sol | pTM624 | 4009 | 4.7 |
| | pTM624 | 5568 | 6.5 |
| | lambda | 127 | 0.16 |
| | lambda | 92 | 0.11 |
| Al$_2$O$_3$ sol | pTM624 | 101,885 | 42 |
| | pTM624 | 94,224 | 39 |
| | lambda | 13,030 | 5.4 |
| | lambda | 17,425 | 7.2 |
| CoO powder | pTM624 | 31,057 | 18 |
| | pTM624 | 33,732 | 20 |
| | lambda | 7493 | 4.4 |
| | lambda | 5956 | 3.5 |
| HfO$_2$ powder | pTM624 | 3602 | 3.0 |
| | pTM624 | 3399 | 2.8 |
| | lambda | 985 | 0.85 |
| | lambda | 1037 | 0.8 |

As can be seen in TABLE 1, each support was useful to form a composition that could distinguish between homologous and non-homologous probe DNA in a hybridization experiment.

EXAMPLE 5

Preparation of ZrO$_2$-PTFE Composite Structure

Part A: ZrO$_2$ Spherules

Porous zirconia spherules (20 grams) prepared as described in EXAMPLE 3 (approximately 20 microns average particle size) were placed in a 250 ml beaker. 3.7 grams of PTFE aqueous dispersion ("Teflon" 30B, solids content of 59.8%) was added in a dropwise manner, while gently hand stirring, as a stiff dough mass was formed. Stirring intensity was increased and continued for 1 minute. The resulting partially fibrillated mass was transferred to a calender and formed into a film. The film was formed between stainless steel calendering rolls (20 cm diameter and 30 cm long) at 50 degrees C, beginning with a 5 millimeter gap and closing the gap on successive passes until a film of approximately 1.25 mm thick was obtained. This film was then folded to form a four (4) layered structure, rotated 90 degrees to the machine direction, and calendered again beginning with a 5 mm nip gap and reducing this gap again until a 1.0 mm composite sheet was formed. The calendered film was then dried at room temperature overnight producing a composite film having a tensile strength of 0.5 megapascal as determined by an Instron tensile testing machine (Instron Corp., Canton, MA).

Part B: ZrO$_2$ Particles

A composite structure was prepared as described in Part A above using ZrO$_2$ particles (20 g, Sigma Chemical Co., Cat. No. Z2500) in place of the porous zirconia spherules.

The resultant composite structure had suitable flexibility and a tensile strength, when measured on a suitable tensile testing device such as an Instron tensile testing machine of at least 0.25 megapascal, with a typical value of 0.5 megapascal. The resultant structure was a white, sheet-like structure of about 0.5 mm (20 mil) thickness, that was flexible and strong, and had a slippery yet rough feel.

EXAMPLE 6

Use of a ZrO$_2$-PTFE Composite Structure in a Southern Blot Hybridization Procedure

Phage lambda DNA was cut with restriction enzyme Bst EII, and pUB110 DNA was cut with Eco RI, and with Hae III. The resultant DNA fragments were electrophoresed in a 1.0% agarose gel, using a Tris acetate buffer (0.45% Tris, 1.14 ml acetic acid per liter). The DNA fragments were electroblotted, using an American Bionetics (Emeryville, CA) Model SBD-1000 transfer system, onto the surface of a PTFE-ZrO$_2$ composite structure prepared as described in EXAMPLES 5A and B. The structure was then sequentially washed five times with 100 ml of 5% potassium acetate, pH 7.0. The structure was then hybridized with either radiolabeled lambda DNA or pUB110 DNA, using the procedure as described in EXAMPLE 2, but with solution volumes increased 100X in each step of the procedure. The amount of radioactive probe hybridized was determined by exposing the membrane to X-ray film at -70 degrees C, for 16 hours. Hybridization was apparent at all appropriate positions on the structure.

EXAMPLE 7

Removal of Previously Hybridized DNA from a ZrO$_2$-PTFE Composite Structure

The PTFE-ZrO$_2$ composite structures used in the DNA hybridization procedure described in EXAMPLE 6, were washed in four sequential solutions of boiling water, 5 minutes per wash, using 0.3 ml/cm$^2$ (300 ml per square foot) of PTFE-ZrO$_2$ membrane. This treatment removed the DNA probe hybridized to the sorbed sequences, without significantly removing the sorbed DNA, as determined by the use of radiolabeled probe DNA molecules. Each 5 minute wash routinely removed more than 95% of the probe used in the previous hybridization. It was found important to use at least four sequential washes, to substantially remove the previously used probe.

EXAMPLE 8

Re-Use of a ZrO$_2$-PTFE Composite Structure

The PTFE-ZrO$_2$ composite structures used in EXAMPLE 6 were treated with 1.0 N NaOH at 80 degrees C for 12 hours, in three sequential washes of 3 hours each. After washing the membranes in distilled water, and then in a weak Tris acetate (10 mM) buffer pH 7.0, the structures were reused in a subsequent DNA sorption procedure, and DNA hybridization assay according to EXAMPLE 6, without any significant impairment of its utility.

13

EP 0 391 608 A2

EXAMPLE 9

Metal Oxide Coated onto a Quartz Surface

ZrO$_2$ was coated onto a monolithic structure of quartz by the following procedure: A quartz microscope slide was dipped into a beaker containing Nyacol™ 100/20 colloidal ZrO$_2$ (Nyacol, Inc.), allowed to air dry, and then heated to 500 degrees C. This procedure was repeated three times with the same slide. The ZrO$_2$-quartz surface was used to sorb DNA, using the procedure described in EXAMPLE 2, except that the quartz-ZrO$_2$ was transferred from one solution to another without a centrifugation step. The sorbed DNA was used in a DNA hybridization assay similar to that described in EXAMPLE 2, except that the ZrO$_2$-quartz slide was transferred from one solution to the next without centrifugation.

Before boiling, it was found that 0.2% of the homologous probe was hybridized, compared to 0.02% of the non-homologous probe. Although the ZrO$_2$-quartz slide surface quenches counting in a scintillation counter, boiling the hybridized probe off the ZrO$_2$ surface increases the number of counts detected by roughly 2-fold.

At the end of the hybridization procedure, if the amount of radioactivity had been determined by a non-destructive means, for example by the exposure of X-ray film, the ZrO$_2$-quartz surface could be washed by adding it to boiling water for 2 minutes, and the immobilized DNA could be rehybridized using a different radiolabeled DNA probe.

Various modifications and alterations of this invention will become apparent to those skilled in the art without departing from the scope and spirit of this invention, and it should be understood that this invention is not to be unduly limited to the illustrative embodiments set forth herein.

## Claims

1. A composition of matter comprising;
   (a) a support comprising a sufficient amount of metal oxide at its surface to sorb nucleic acid,
   (b) nucleic acid sorbed to at least a portion of the available surface of said support in a manner such that said nucleic acid substantially retains biological accessibility and reactivity.

2. A composition according to claim 1 further comprising blocking agent bound to substantially the remainder of the available surface of said support in a manner that substantially precludes the ability of the support to sorb additional nucleic acid, wherein said blocking agent is selected from the group consisting of organic or inorganic molecules providing phosphate, phosphonate, pyrophosphate, carboxylate, sulfate and fluoride groups.

3. A composition according to claim 1 wherein said support is in a form selected from the group consisting of particles, fibers, and monolithic bodies, and wherein said metal oxide is selected from the group consisting of magnesium, aluminum, calcium, titanium, manganese, iron, cobalt, nickel, zinc, yttrium, zirconium, lanthanum, and hafnium.

4. A composition according to claim 3 wherein said support is in the form of a particle and said particle is used in a conformation selected from the group consisting of suspensions, coatings, and composites.

5. A composition according to claim 4 wherein said conformation is a composite provided in the form of a web of PTFE.

6. A composition according to claim 3 wherein said metal oxide is zirconium or aluminum.

7. A method of preparing a composition of matter comprising the steps of:
   (a) providing a support comprising an amount of metal oxide at its surface sufficient to sorb nucleic acid, and
   (b) sorbing nucleic acid to at least a portion of the available surface of said support in a manner that substantially retains biological accessibility and reactivity of the nucleic acid.

8. A method according to claim 16 further comprising the step of binding blocking agent to substantially the remainder of the available surface of said support in a manner that substantially precludes the ability of the support to sorb additional nucleic acid.

9. A method of hybridizing, labeling, and/or sequencing nucleic acid comprising the steps of
   (a) providing a composition comprising
      (i) a support comprising an amount of metal oxide sufficient to sorb nucleic acid,

14

(ii) nucleic acid sorbed to at least a portion of the available surface of said support in a manner such that said nucleic acid substantially retains biological accessibility and reactivity, and

(iii) blocking agent bound to substantially the remainder of the available surface of said support in a manner that substantially precludes the ability of the support to sorb additional nucleic acid, and

(b) hybridizing, labeling, or sequencing said sorbed nucleic acid.